(19) Europäisches Patentamt

European Patent Office

Office européen des brevets

(11) **EP 3 653 109 A1**

(12) **EUROPEAN PATENT APPLICATION**
published in accordance with Art. 153(4) EPC

(43) Date of publication:
**20.05.2020 Bulletin 2020/21**

(21) Application number: **18831414.0**

(22) Date of filing: **05.07.2018**

(51) Int Cl.:
**A61B 5/00** *(2006.01)*        **G16H 10/00** *(2018.01)*

(86) International application number:
**PCT/JP2018/025539**

(87) International publication number:
**WO 2019/013094 (17.01.2019 Gazette 2019/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.07.2017 JP 2017138390**

(71) Applicant: **Public University Corporation Yokohama City University**
**Yokohama-shi, Kanagawa 236-0027 (JP)**

(72) Inventor: **TAKAKI, Shunsuke**
**Yokohama-shi**
**Kanagawa 236-0004 (JP)**

(74) Representative: **TBK**
**Bavariaring 4-6**
**80336 München (DE)**

(54) **AGGRAVATION ESTIMATION DEVICE AND AGGRAVATION ESTIMATION PROGRAM**

(57)    To provide an aggravation estimation device and an aggravation estimation program for accurately estimating aggravation of a patient as a subject based on various types of information regarding the patient as the subject. A computer terminal (10) implements the functions of: generating a static scoring model in which first and second variables based on patient information extracted from a database (40) correspond to vertical and horizontal axes, respectively; acquiring types of patient information regarding a patient (30) per unit time, and deriving the variables from the acquired patient information to successively draw plots on the static scoring model; and estimating points in time at which the plots are drawn by the function in a specific region of the static scoring model as the timing at which the subject is aggravated, wherein at least one of the variables is an index derived by using the types of patient information.

FIG. 1

Printed by Jouve, 75001 PARIS (FR)

**EP 3 653 109 A1**

**Description**

TECHNICAL FIELD

The present invention relates to an aggravation estimation device and an aggravation estimation program.

BACKGROUND ART

Recently, in the medical field, there have been researches and development carried out for utilizing biological information of a certain patient measured therefrom not only for treatment, diagnosis, and the like of the patient but also for treatment, diagnosis, and the like of other patients as well.
Examples of such kind of technologies include the following Patent Document 1.
In Patent Document 1, a system is disclosed that generates an alarm when a patient is aggravated. The system utilizes various kinds of physiological scoring systems or physiological parameters such as early warning scores (EWS), modified early warning scores (MEWS), and vital signs indexes (VIX).
In Patent Document 1, it is disclosed to plot actually measured values of the heart rate and respiratory rate as part of information used for the early warning scores on a two-dimensional model that takes the heart rate as the vertical axis and the respiratory rate as the horizontal axis, and to generate an alarm when the plot is displayed in a specific region of the two-dimensional model.

CITATION LIST

PATENT DOCUMENTS

Patent Document 1: National Publication of International Patent Application No. 2014-510603

SUMMARY OF THE INVENTION

PROBLEM TO BE SOLVED BY THE INVENTION

The two-dimensional model disclosed in Patent Document 1 is effective in terms of making it easy to make intuitive determination by visually displaying the early warning score. However, only the heart rate and the respiratory rate are selectively used in spite of the fact that the early warning score is an index for determining the seriousness of the patient by adding parameters such as a systolic blood pressure, a degree of oxygen saturation, and a body temperature to the heart rate and the respiratory rate, so that there is a concern that the validity thereof may become insufficient.
The present invention, which is designed in view of the foregoing problem, provides an aggravation estimation device and an aggravation estimation program for accurately estimating aggravation of a patient as a subject based on various types of information regarding the patient as the subject.

MEANS FOR SOLVING THE PROBLEM

The present invention provides an aggravation estimation device, including: a model generation unit that extracts, from a database accumulating a plurality of types of patient information acquired chronologically for a plurality of patients, patient information acquired in a predetermined period including at least a period before a point in time at which each of the patients is aggravated, derives a first variable and a second variable from the extracted patient information, and generates a static scoring model as a two-dimensional model in which the derived first variable and second variable correspond to a vertical axis and a horizontal axis, respectively; a scoring unit that acquires the plurality of types of patient information regarding the patient as a subject per unit time, and derives the first variable and the second variable from the acquired patient information to successively draw plots on the static scoring model; and an aggravation estimation unit that estimates at least part of points in time at which the plots are drawn by the scoring unit in a specific region that is part of a two-dimensional region of the static scoring model as a timing at which the subject is aggravated, wherein at least one of the first variable and the second variable is an index derived by using the plurality of types of patient information.
The present invention provides an aggravation estimation program for causing a computer to execute: a model generation unit that extracts, from a database accumulating a plurality of types of patient information acquired chronologically for a plurality of patients, patient information acquired in a predetermined period including at least a period before a point in time at which each of the patients is aggravated, derives a first variable and a second variable from the extracted patient information, and generates a static scoring model as a two-dimensional model in which the derived first variable and second variable correspond to a vertical axis and a horizontal axis, respectively; a scoring unit that acquires the plurality of types of patient information regarding the patient as a subject per unit time, and derives the first variable and the second variable from the acquired patient information to successively draw plots on the static scoring model; and an aggravation estimation unit that estimates at least part of points in time at which the plots are drawn by the scoring unit in a specific region that is part of a two-dimensional region of the static scoring model as a timing at which the subject is aggravated, wherein at least one of the first variable and the second variable is an index derived by using the plurality of types of patient information.

In the present invention, at least one of the first variable and the second variable corresponding to the vertical axis and the horizontal axis of the two-dimensional model (static scoring model) to be scored is the index derived by using a plurality of types of patient information. Accordingly, it is possible with the present invention to plot the patient information with higher validity compared to that of the conventional technique on the two-dimensional model and to accurately estimate the timing at which the subject is aggravated.

EFFECT OF THE INVENTION

The present invention provides the aggravation estimation device and the aggravation estimation program for accurately estimating aggravation of a patient as a subject based on various types of information regarding the patient as the subject.

BRIEF DESCRIPTION OF THE DRAWINGS

Fig. 1 is a system configuration diagram for implementing the present invention.
Fig. 2 is a chart showing a specific example of a display screen of a computer terminal.

DESCRIPTION OF EMBODIMENTS

Hereinafter, an embodiment of the present invention will be described by referring to the accompanying drawings. Note that the same reference signs are applied to the same structural elements all through the drawings, and explanations thereof will not be repeated.

<Aggravation Prediction Device according to Present Invention>

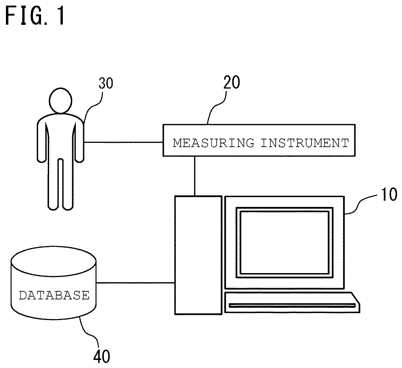

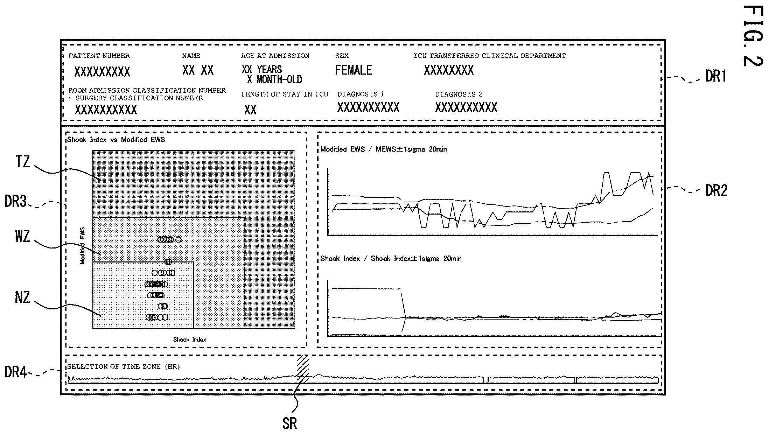

First, an aggravation prediction device according to the present invention will be described by using Fig. 1 and Fig. 2.
Fig. 1 is a system configuration diagram for implementing the present invention. Fig. 2 is a chart showing a specific example of a display screen of a computer terminal.
The aggravation prediction device according to the present invention is implemented by a computer terminal 10 to which dedicated application software (an aggravation prediction program according to the present invention) is installed, for example.
The computer terminal 10 is capable of achieving each function to be described later by executing processing related to the application software, and includes hardware resources required for achieving the function. Specifically, the hardware resources herein may be a CPU and a memory built in the computer terminal 10, an input device for receiving operation input from a user, and an output device for outputting screens, sound, and the like required for implementing operations of the user and each of the functions, for example.
The computer terminal 10 is capable of implementing at least the following functions.
The first function is to extract, from a database 40, patient information acquired in a predetermined period including at least a period before the point in time at which each patient is aggravated, derive a first variable and a second variable from the extracted patient information, and generate a static scoring model as a two-dimensional model in which the derived first variable and second variable correspond to the vertical axis and the horizontal axis, respectively (hereinafter, referred to as a model generation unit).
The second function is to acquire a plurality of types of patient information regarding a patient 30 as a subject per unit time, and derive the first variable and the second variable from the acquired patient information to successively draw plots on the static scoring model (hereinafter, referred to as a scoring unit).
The third function is to estimate, as the timing at which the subject is aggravated, at least part of points in time at which the plots are drawn by the scoring unit in a specific region as part of a two-dimensional region in the static scoring model (hereinafter, referred to as an aggravation estimation unit).

The database 40 accumulates a plurality of types of patient information acquired chronologically for a plurality of patients. The database 40 may be a dedicated database provided to introduce the present invention or may be a general database provided for a system (for example, an electronic medical record system) introduced separately from the present invention.

The patient information accumulated in the database 40 includes information regarding personal attributes of the patient (for example, name of the patient, sex, age, name of disease, identification number of the patient, and the like) and biological information of the patient (for example, the body temperature, the heart rate, the respiratory rate, the degree of oxygen saturation, the blood pressure, and the like). Note here that the blood pressure may correspond to any of a systolic blood pressure, a diastolic blood pressure, and a mean blood pressure.

The static scoring model is a two-dimensional model that takes, as the vertical axis and the horizontal axis, the first variable and the second variable derivable from the patient information accumulated in the database 40. Note that the static scoring model according to the present invention is defined as "static" with the fact that the range of the specific region to be the criteria for performing processing by the aggravation estimation unit after being generated by the model generation unit is invariable. Therefore, it is possible to change the specific region of the static scoring model to be variable depending on various conditions used by the model generation unit and to dynamically change display modes of the static scoring model displayed on the display screen of the computer terminal 10.

Note here that at least one of the first variable and the second variable is an index derived by using a plurality of types of patient information. For example, in a case shown in Fig. 2, the first variable corresponding to the horizontal axis is a shock index, and the second variable corresponding to the vertical axis is a modified early warning score. Instead of such case, either one of the first variable or the second variable may be a single type of patient information (biological information) such as the heart rate, the respiratory rate, or the systolic blood pressure.

As shown in Fig. 2, the display screen of the computer terminal 10 may be divided mainly into four parts. In Fig. 2, those four parts are shown by being surrounded by broken lines. Note that those broken lines are not actually displayed.

A patient attribute display part DR1 located in the uppermost part is a region that displays information related to personal attributes of a subject (patient 30). In the embodiment, displayed in the patient attribute display part DR1 are the identification number of the patient, name, age at the time of admission, sex, transferred clinical department, room admission classification number or surgery classification number, length of stay, and names of diagnosed diseases.

While it is configured to be capable of displaying a plurality of names of diagnosed diseases, Fig. 2 shows a state where only one type is being displayed.

An index transition display part DR2 located on the right side of the middle part is a region that displays chronological changes of the modified early warning score and the shock index. In the display of the index transition display part DR2 of the embodiment, the vertical axis is the modified early warning score or the shock index, and the horizontal axis is the time. Transitions of the modified early warning score and the shock index are shown with solid lines, transitions of values in which $+1\sigma$ is added to each thereof are shown with long dashed short dashed lines, and transitions of values in which $-1\sigma$ is added to each thereof are shown with long dashed double-short dashed lines. Note here that "$\sigma$" is a standard deviation of the modified early warning score or the shock index.

By observing chronological changes of the modified early warning score and the shock index described above, it is possible to predict whether the condition of the patient will get worse or better than the current condition. Further, through analyzing variations in the modified early warning score and the shock index by referring also to the values with $\pm 1\sigma$ added thereto, it is possible to predict the condition of the patient with still higher accuracy.

Note that an upper limit is set for the horizontal axis in the index transition display part DR2. For example, in Fig. 2, 20 minutes is set as the upper limit, and chronological changes of the modified early warning score and the shock index are displayed within that range. The time zone displayed in the index transition display part DR2 is displayed in a time zone display part DR4 to be described later.

The model display part DR3 located on the left side of the middle part is a region that displays the static scoring model. The static scoring model of the embodiment is sectioned into three regions that are a stable condition zone NZ, a warning zone WZ, and a terminal zone TZ. The stable condition zone NZ is a region showing that the condition of the patient 30 is most stable, and the terminal zone TZ is a region showing that the condition of the patient 30 is most critical. The computer terminal 10 estimates the plotting time to the terminal zone TZ as the timing at which the subject is aggravated. That is, the terminal zone TZ corresponds to the specific region described above.

Even when a plot is drawn in the terminal zone TZ, it is not necessary to immediately estimate as aggravation. It is because the possibility of aggravation is considered to be low when the condition is recovered immediately even if the condition of the patient 30 temporarily changes and a plot is drawn in the terminal zone TZ. Therefore, when plots are drawn in the terminal zone TZ over a specific length of time (when a specific number of plots are continuously drawn in the terminal zone TZ), it then may be estimated as aggravation of the patient 30.

While those three regions are shown to be distinguishable in the embodiment for the sake of explanations, the computer terminal 10 does not necessarily need to display each of the regions to be distinguishable.

The time zone display part DR4 located in the lowermost part is a region that displays which part among the whole time the time zone being displayed at that point in time in the index transition display part DR2 corresponds to. More specifically, a selected region SR out of the whole time (hatched part in the time zone display part DR4) corresponds to the time zone displayed in the index transition display part DR2.

Various types of information displayed on the display screen of the computer terminal 10 as described above are generated based on each piece of data accumulated in the database 40 or generated based on biological information (vital signs) of the patient 30 chronologically measured by a measuring instrument 20. Thereby, "visualization" of the state (condition) of the whole body of the patient 30 may be implemented.

While the time interval for measuring the biological information by the measuring instrument 20 (time interval at which the computer terminal 10 acquires the measurement result from the measuring instrument 20) may be set freely, it is preferable to be set in the order of minutes or less in order to estimate aggravation timing of the patient 30 with specific accuracy. Note here that the order of minutes or less means that the time interval for the computer terminal 10 to acquire is a range not exceeding 1 hour (60 minutes), and it is more preferable to be a range not exceeding 10 minutes. In the embodiment, the time interval for measuring the biological information by the measuring instrument 20 is set as 1-minute interval in principle.

As described above, the present invention is capable of accurately and automatically estimating aggravation of the patient 30 through performing computer processing using the static scoring model generated based on a great amount of past patient information and appropriately notifying the medical personnel about the timing that requires therapeutic intervention.

Hereinafter, each feature of the present invention will be described in detail.

<First Variable and Second Variable>

It is preferable to employ the first variable and the second variable having at least one type in common in the patient information used for deriving the first variable and in the patient information used for deriving the second variable. Thereby, the first and second variables come to have a specific correlation with each other, so that behavior of the plots drawn in the static scoring model becomes more significant for estimating aggravation of the subject.

For example, in the case of the static scoring model shown in Fig. 2, the first variable is the shock index derived by dividing the heart rate by the systolic blood pressure, and the second variable is the modified early warning score derived based on the heart rate, the systolic blood pressure, the respiratory rate, the degree of oxygen saturation, and the body temperature.

A method for deriving the modified early warning score will be described. The following table is used for deriving the modified early warning score.

[Table 1]

|  | 3 | 2 | 1 | 0 | 1 | 2 | 3 |
|---|---|---|---|---|---|---|---|
| RR | $\leqq$8 |  | 9-11 | 12-20 |  | 21-24 | $\geqq$25 |
| Sp02 | $\leqq$91 | 92-93 | 94-95 | $\geqq$96 |  |  |  |
| BT | $\leqq$35.0 |  | 35.1-36.0 | 36.1-38.0 | 38.1-39.0 | $\leqq$39.1 |  |
| ABPs | $\leqq$90 | 91-100 | 101-110 | 111-219 |  |  | $\geqq$220 |
| HR | $\leqq$40 |  | 41-50 | 51-90 | 91-110 | 111-130 | $\geqq$131 |

The computer terminal 10 acquires scores of each of items by collating the respiratory rate (RR), the degree of oxygen saturation (SpO2), the body temperature (BT), the systolic blood pressure (ABPs), and the heart rate (HR) acquired per unit time with the table shown above. Then, the total value of the acquired scores is the modified early warning score. Therefore, when the patient information of the patient 30 at a certain point in time indicates that the respiratory rate is 18 times, the degree of oxygen saturation is 95%, the body temperature is 38.5 degrees Celsius, the systolic blood pressure is 100 mmHg, and the heart rate is 100 times, for example, the modified early warning score is calculated as 5 (= 0+1+1+2+1).

Because of the above manner it is derived, the modified early warning score is derived as an integer of 0 (zero) or larger.

Alternatively, in the embodiment of the present invention, an intervention prediction score (IPS) may be used for the second variable instead of the modified early warning score while the shock index is used for the first variable. Note here that the intervention prediction score is an index uniquely built by the inventor of the present invention, and derived based on the age, the diastolic blood pressure, the body temperature, the heart rate, the respiratory rate, and the degree of oxygen saturation. Details of the intervention prediction score will be described later.

<How to Define Terminal Zone TZ>

Subsequently, the way of defining the terminal zone TZ in the static scoring model will be described. For defining the terminal zone TZ, it is necessary to define a threshold value of the shock index (first variable) corresponding to the horizontal axis and a threshold value of the modified early warning score (second variable), respectively.

It is preferable for the computer terminal 10 (model generation unit) to search the database for a plurality of patients 40 by using at least one type among the patient information related to the subject as a key, and define the terminal zone TZ (threshold values of the first variable and the second variable) in the static scoring model based on the patient information related to a plurality of patients searched for. It is because aggravation of the subject may be estimated more accurately by making analyses targeted on the patients having attributes and disease close to the subject.

In the explanations below, 18 patients suffering from rheumatism/blood disease and urgently admitted to an intensive care unit were acquired from the database 40 to be the target of analysis for setting the terminal zone TZ in the static scoring model.

Specifically, acquired was the patient information of those 18 patients at the point in time at which medical intervention such as tracheal intubation and administration of vasopressor was carried out and 30 minutes before and after that point in time (hereinafter, the patient information in this 60-minute period is referred to as intervention group data).

Further, 24 hours later from the point in time at which the medical intervention was carried out was set as a reference time, and the patient information of the same 18 patients was acquired 30 minutes before and after the reference time (hereinafter, the patient information in this 60-minute period is referred to as stable group data).

Note that it is possible to change as appropriate what point in time is set as the reference or the patient information in the period of how long from the point in time of the reference is taken as the target.

Missing data, data at the time of taking a blood sample, and the like were excluded from the target of analysis among the intervention group data and the stable group data acquired in the manner described above, and 470 points were used for the intervention group data and 894 points were used for the stable group data.

The following table shows the sensitivity and the specificity acquired according to the modified early warning score calculated based on the data. Note here that the sensitivity and the specificity are quantitative indexes that show how accurately the examination is able to determine presence of a specific disease.

The numerical values written in the right columns of the sensitivity column and the specificity column are the confidence interval (CI) of the sensitivity or the specificity with a confidence level of 95%. The numerical values written in the rightmost columns in each of the rows are likelihood ratios of the sensitivity and the specificity.

[Table 2]

| Cutoff | Sensitivity% | 95% CI | Specificity% | 95% CI | Likelihoo d ratio |
|---|---|---|---|---|---|
| < 0.5000 | 5.145 | 3.791% to 6.804% | 99.79 | 98.82% to 99.99% | 24.18 |
| < 1.500 | 28.08 | 25.15% to 31.15% | 98.30 | 96.67% to 99.26% | 16.49 |
| < 2.500 | 44.30 | 41.01% to 47.62% | 95.96 | 93.76% to 97.55% | 10.96 |
| < 3.500 | 64.65 | 61.42% to 67.79% | 85.74 | 82.25% to 88.78% | 4.535 |
| *< 4.500* | *87.25* | *84.88% to 89.37%* | *75.53* | *71.39% to 79.35%* | *3.566* |
| *< 5.500* | *91.83* | *89.84% to 93.55%* | *59.15* | *54.55% to 63.63%* | *2.248* |
| < 6.500 | 99.66 | 99.02% to 99.93% | 43.83 | 39.29% to 48.45% | 1.774 |
| < 7.500 | 100.0 | 99.59% to 100.0% | 30.21 | 26.09% to 34.59% | 1.433 |
| < 8.500 | 100.0 | 99.59% to 100.0% | 15.11 | 11.99% to 18.67% | 1.178 |
| < 9.500 | 100.0 | 99.59% to 100.0% | 6.809 | 4.703% to 9.476% | 1.073 |
| < 10.50 | 100.0 | 99.59% to 100.0% | 3.404 | 1.958% to 5.470% | 1.035 |
| < 11.50 | 100.0 | 99.59% to 100.0% | 1.702 | 0.7376% to 3.326% | 1.017 |
| < 12.50 | 100.0 | 99.59% to 100.0% | 0.8511 | 0.2324% to 2.165% | 1.009 |
| < 13.50 | 100.0 | 99.59% to 100.0% | 0.4255 | 0.05158% to 1.529% | 1.004 |

Looking into the case where the threshold value is set as 4.5 (Cut off < 4.5) in the above table, 87.25% of the stable group data is included when MEWS < 4.5 while 75.53% of intervention group data is included when MEWS > 4.5. That is, it may be interpreted that intervention is necessary in 75% of circumstances when MEWS exceeds 4.5.

Further, looking into the case where the threshold value is set as 5.5 (Cut off < 5.5) in the above table, 91.83% of the stable group data is included when MEWS < 5.5 while 59.15% of intervention group data is included when MEWS > 5.5. That is, it may be interpreted that intervention is necessary in 59% of circumstances when MEWS exceeds 5.5.

As described above, the modified early warning score takes the value derived as an integer of 0 (zero) or larger, so that it is determined appropriate to set the threshold value regarding the modified early warning score to 5 based on the analysis result.

Note that this threshold value is simply a specific example and may change depending on the disease and the like of the subject and may change by altering the range of the patient information as the analysis target, so that the threshold value in the embodiment of the present invention is not limited thereto.

The method described above may also be applied when setting the threshold value of the shock index. Therefore, by combining the threshold value of the shock index and the threshold value of the modified early warning score derived independently, the computer terminal 10 is able to set the terminal zone TZ.

<Construction of Intervention Prediction Score>

As described above, for the second variable, it is possible to use the intervention prediction score instead of the modified early warning score.

As the candidates for the variables used for the intervention prediction score, there may be background data such as the age, sex, past medical history and the like of the patient, medicine used for treatment, evaluation items of sedation/analgesia, patient information that may be acquired by the measuring instrument 20, and numerical value data acquired by analyzing images captured by a camera or the like, for example.

Those variables are compared with the data in the aggravated state and the data of the stable stage. As a method for comparison and investigation, it is possible to apply linear regression analysis, logistic regression analysis, nonlinear regression analysis, machine learning, and the like.

In the embodiment, as a specific example, the intervention group data used for setting the threshold value described above is treated as "data in aggravated state", and the stable group data is treated as "data of stable stage".

By analyzing the diastolic blood pressure (ABPd), the mean blood pressure (ABPm), the systolic blood pressure (ABPs), the age (Age), the body temperature (BT), the heart rate (HR), the respiratory rate (RR), the sex (Sex), and the degree of oxygen saturation (SpO2) used as explanatory variables in those data groups, mutual correlations were acquired. Note that the correlations of the total of the early warning score (EWS) with respect to the above described explanatory variables were also acquired for reference.

The values written in each column of the following table show correlations of each of the explanatory variables, and the higher the value is, the stronger the correlation is. In this case, the diastolic blood pressure, the mean blood pressure, and the systolic blood pressure are strongly correlated to each other, so that part thereof may be excluded from variable options regarding the intervention prediction score. In the embodiment, the intervention prediction score is constructed by using only the diastolic blood pressure among those.

[Table 3]

| ABPd | ABPm | ABPs | Age | BT | EWS total | HR | RR | Sex | Sp02 |
|------|------|------|-----|-----|-----------|------|------|------|------|
| 27.6 | 44.2 | 6.70 | 1.42 | 1.46 | 5.23 | 2.14 | 2.87 | 1.24 | 2.35 |

The intervention prediction score was set as 100 points for the above described intervention group data while the intervention prediction score was set as 0 point for the above described stable group data, and multiple regression analysis was carried out for those continuous variables. As a result, the following prediction expression having the age (Age), the diastolic blood pressure (ABPd), the body temperature (BT), the heart rate (HR), the respiratory rate (RR), and the degree of oxygen saturation (SpO2) as the variables is derived.

[Expression 1]

$$IPS = -404.7 + (0.71 \times Age) + (-1.00 \times ABPd) + (13.6 \times BT) + (0.92 \times HR) + (1.95 \times RR) + (-1.82 \times SpO2)$$

When the expression above is used, the point in time at which the value of the intervention prediction score (IPS) exceeds 100 or the point in time at which the value is close to 100 may be estimated as the medical intervention timing, that is, as the timing at which the subject is aggravated. When the computer terminal 10 calculates the intervention prediction score by using the above expression, the solution thereof takes a larger value compared to that of the modified early warning score and, further, it is possible to acquire the solution to decimal places. Therefore, the plot pattern on the static scoring model using the intervention prediction score for the second variable fluctuates chronologically more minutely compared to the case of using the modified early warning score for the second variable. Therefore, estimating the aggravation timing of the subject by using the intervention prediction score results in achieving higher accuracy compared to estimating it by using the modified early warning score. Because the age factor is employed in addition to the items of the modified early warning score, it is predicted that sensitivity and specificity for the elderly are improved so that the usefulness in the clinical practice considered to be increased.

Note that selections of each of the variables in the expression above and the content of the prediction expression may change when the data to be the target of analysis is changed, so that the embodiment of the present invention is not limited thereto.

<Modification Example of the Present Invention>

While the present invention has been described heretofore with reference to the embodiment, the present invention is not limited to the embodiment described above but includes various kinds of changes, modifications, and the like as long as the object of the present invention is implemented therewith.

In the above embodiment, while the display screen of the computer terminal 10 shown in Fig. 2 is illustrated to include the patient attribute display part DR1, the index transition display part DR2, and the time zone display part DR4 in addition to the model display part DR3 that displays the static scoring model, the embodiment of the display screen is not limited thereto.

For example, the display screen may be configured only by a display corresponding to the model display part DR3 or may be configured with another display not shown in Fig. 2.

While it has been described to individually acquire the threshold value of the first variable and the threshold value of the second variable as the method for setting the specific region (terminal zone TZ) in the above embodiment, the embodiment of the present invention is not limited thereto.

For example, it is possible to use a method for setting the specific region by relating the first variable with the second variable (method with which the threshold value of the specific region is derived by a function using both the first variable and the second variable as the variables).

The embodiment includes the following technical ideas.

(1) An aggravation estimation device, including: a model generation unit that extracts, from a database accumulating a plurality of types of patient information acquired chronologically for a plurality of patients, patient information acquired in a predetermined period including at least a period before a point in time at which each of the patients is aggravated, derives a first variable and a second variable from the extracted patient information, and generates a static scoring model as a two-dimensional model in which the derived first variable and second variable correspond to a vertical axis and a horizontal axis, respectively; a scoring unit that acquires the plurality of types of patient information regarding the patient as a subject per unit time, and derives the first variable and the second variable from the acquired patient information to successively draw plots on the static scoring model; and an aggravation estimation unit that estimates at least part of points in time at which the plots are drawn by the scoring unit in a specific region that is part of a two-dimensional region of the static scoring model as a timing at which the subject is aggravated, wherein at least one of the first variable and the second variable is an index derived by using the plurality of types of patient information.

(2) The aggravation estimation device according to (1), wherein the patient information used for deriving the first variable and the patient information used for deriving the second variable have at least one type in common.

(3) The aggravation estimation device according to (2), wherein: the first variable is an index derived by dividing a heart rate by a systolic blood pressure; and the second variable is an index derived based on the heart rate, the systolic blood pressure, a respiratory rate, a degree of oxygen saturation, and a body temperature.

(4) The aggravation estimation device according to (2), wherein: the first variable is an index derived by dividing a heart rate by a systolic blood pressure; and the second variable is an index derived based on age, a diastolic blood pressure, a body temperature, the heart rate, a respiratory rate, and a degree of oxygen saturation.

(5) The aggravation estimation device according to any one of (1) to (4), wherein the model generation unit searches the database for a plurality of patients by using at least one type of the patient information related to the subject as a key, and defines the specific region of the static scoring model based on the patient information of the plurality of patients searched for.

(6) An aggravation estimation program for causing a computer to execute: a model generation unit that extracts, from a database accumulating a plurality of types of patient information acquired chronologically for a plurality of patients, patient information acquired in a predetermined period including at least a period before a point in time at which each of the patients is aggravated, derives a first variable and a second variable from the extracted patient information, and generates a static scoring model as a two-dimensional model in which the derived first variable and second variable correspond to a vertical axis and a horizontal axis, respectively; a scoring unit that acquires the plurality of types of patient information regarding the patient as a subject per unit time, and derives the first variable and the second variable from the acquired patient information to successively draw plots on the static scoring model; and an aggravation estimation unit that estimates at least part of points in time at which the plots are drawn by the scoring unit in a specific region that is part of a two-dimensional region of the static scoring model as a timing at which the subject is aggravated, wherein at least one of the first variable and the second variable is an index derived by using the plurality of types of patient information.

This application claims the benefit of priority to Japanese Patent Application No. 2017-138390, filed on July 14, 2017, which is hereby incorporated by reference in its entirety.

REFERENCE SIGNS LIST

| 10 | Computer terminal |
| 20 | Measuring instrument |
| 30 | Patient |
| 40 | Database |

**Claims**

1. An aggravation estimation device comprising:

    a model generation unit that extracts, from a database accumulating a plurality of types of patient information acquired chronologically for a plurality of patients, patient information acquired in a predetermined period including at least a period before a point in time at which each of the patients is aggravated, derives a first variable and a second variable from the extracted patient information, and generates a static scoring model as a two-dimensional model in which the derived first variable and second variable correspond to a vertical axis and a horizontal axis, respectively;
    a scoring unit that acquires the plurality of types of patient information regarding the patient as a subject per unit time, and derives the first variable and the second variable from the acquired patient information to successively draw plots on the static scoring model; and
    an aggravation estimation unit that estimates at least part of points in time at which the plots are drawn by the scoring unit in a specific region that is part of a two-dimensional region of the static scoring model as a timing at which the subject is aggravated, wherein
    at least one of the first variable and the second variable is an index derived by using the plurality of types of patient information.

2. The aggravation estimation device according to claim 1, wherein the patient information used for deriving the first variable and the patient information used for deriving the second variable have at least one type in common.

3. The aggravation estimation device according to claim 2, wherein:

    the first variable is an index derived by dividing a heart rate by a systolic blood pressure; and
    the second variable is an index derived based on the heart rate, the systolic blood pressure, a respiratory rate, a degree of oxygen saturation, and a body temperature.

4. The aggravation estimation device according to claim 2, wherein:

    the first variable is an index derived by dividing a heart rate by a systolic blood pressure; and
    the second variable is an index derived based on age, a diastolic blood pressure, a body temperature, the heart rate, a respiratory rate, and a degree of oxygen saturation.

5. The aggravation estimation device according to any one of claims 1 to 4, wherein the model generation unit searches the database for a plurality of patients by using at least one type of the patient information related to the subject as a key, and defines the specific region of the static scoring model based on the patient information of the plurality of patients searched for.

6. An aggravation estimation program for causing a computer to execute:

a model generation unit that extracts, from a database accumulating a plurality of types of patient information acquired chronologically for a plurality of patients, patient information acquired in a predetermined period including at least a period before a point in time at which each of the patients is aggravated, derives a first variable and a second variable from the extracted patient information, and generates a static scoring model as a two-dimensional model in which the derived first variable and second variable correspond to a vertical axis and a horizontal axis, respectively;
a scoring unit that acquires the plurality of types of patient information regarding the patient as a subject per unit time, and derives the first variable and the second variable from the acquired patient information to successively draw plots on the static scoring model; and
an aggravation estimation unit that estimates at least part of points in time at which the plots are drawn by the scoring unit in a specific region that is part of a two-dimensional region of the static scoring model as a timing at which the subject is aggravated, wherein
at least one of the first variable and the second variable is an index derived by using the plurality of types of patient information.

FIG. 1

FIG. 2

EP 3 653 109 A1

12

## INTERNATIONAL SEARCH REPORT

| International application No. |
| --- |
| PCT/JP2018/025539 |

### A. CLASSIFICATION OF SUBJECT MATTER
Int.Cl. A61B5/00(2006.01)i, G16H10/00(2018.01)i

According to International Patent Classification (IPC) or to both national classification and IPC

### B. FIELDS SEARCHED

Minimum documentation searched (classification system followed by classification symbols)
Int.Cl. A61B5/00, G16H10/00

Documentation searched other than minimum documentation to the extent that such documents are included in the fields searched
| | |
| --- | --- |
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| A | JP 2008-99876 A (CROSSWELL CO., LTD.) 01 May 2008, abstract, paragraphs [0008]–[0009], [0025], [0051], [0056]–[0058], fig. 9 (Family: none) | 1–6 |
| A | JP 2014-510603 A (KONINKLIJKE PHILIPS N.V.) 01 May 2014, abstract, paragraphs [0040]–[0044], fig. 11–12 & US 9189941 B2, abstract, column 7, line 33 to column 8, line 39, fig. 11–12 & WO 2012/140547 A1 & CN 103476328 A | 1–6 |

☒ Further documents are listed in the continuation of Box C.       ☐ See patent family annex.

| | | |
| --- | --- | --- |
| * | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
| --- | --- |
| 07 September 2018 (07.09.2018) | 18 September 2018 (18.09.2018) |

| Name and mailing address of the ISA/ | Authorized officer |
| --- | --- |
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

## INTERNATIONAL SEARCH REPORT

| | International application No. |
|---|---|
| | PCT/JP2018/025539 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2010-172365 A (CROSSWELL CO., LTD.) 12 August 2010, abstract, paragraphs [0022], [0044]-[0068], fig. 6-13 (Family: none) | 1-6 |
| A | WO 2016/079654 A1 (KONINKLIJKE PHILIPS N. V.) 26 May 2016, abstract, page 10, line 9 to page 11, line 23, fig. 3A-3B & JP 2018-506759 A, abstract, paragraphs [0025]-[0026], [0039]-[0042], fig. 3A-3B & US 2017/0360379 A1 & CN 107106024 A | 1-6 |
| A | JP 2014-140521 A (PANASONIC HEALTHCARE CO., LTD.) 07 August 2014, abstract, paragraphs [0011], [0030]-[0044], fig. 5-6 (Family: none) | 1-6 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014510603 A

- JP 2017138390 A